(19) Europäisches Patentamt
European Patent Office
Office européen des brevets

(11) Veröffentlichungsnummer: **0 261 605 A1**

(12) # EUROPÄISCHE PATENTANMELDUNG

(21) Anmeldenummer: **87113690.9**

(22) Anmeldetag: **18.09.87**

(51) Int. Cl.4: **C07C 93/04**, C07C 119/042, C07C 118/00

(30) Priorität: **20.09.86 DE 3632007**

(43) Veröffentlichungstag der Anmeldung: **30.03.88 Patentblatt 88/13**

(84) Benannte Vertragsstaaten: **BE CH DE ES FR GB IT LI NL**

(71) Anmelder: **BASF Aktiengesellschaft**
**Carl-Bosch-Strasse 38**
**D-6700 Ludwigshafen(DE)**

(72) Erfinder: **Merger, Franz, Dr.**
**Max-Slevogt-Strasse 25**
**D-6710 Frankenthal(DE)**
Erfinder: **Liebe, Joerg, Dr.**
**Hans-Purrmann-Strasse 10b**
**D-6710 Frankenthal(DE)**

(54) **In 2-Stellung substituierte 1,5-Diaminopentane.**

(57) In 2-Stellung substituierte 1,5-Diaminopentane (I)

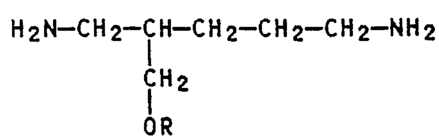

(I)

(R = $C_1$-$C_{20}$-Alkyl; $C_3$-$C_{20}$-Oxoalkyl; $C_2$-$C_{20}$-Alkenyl; $C_5$-$C_{12}$-Cycloalkyl; $C_7$-$C_{20}$-Aralkyl), sowie Verfahren zur Herstellung dieser Verbindungen durch Hydrierung der entsprechenden Dinitrile. Die Verbindungen (I) sind u.a. zur Herstellung der entsprechenden Diisocyanate geeignet.

EP 0 261 605 A1

# In 2-Stellung substituierte 1,5-Diaminopentane

Die Erfindung betrifft neue, in 2-Stellung substituierte 1,5-Diaminopentane, ein Verfahren zu ihrer Herstellung und deren Verwendung zur Herstellung der entsprechenden Diisocyanate.

Der Erfindung lag die Aufgabe zugrunde, neue, als Zwischenprodukte wertvolle Diamine, sowie ein Verfahren zu ihrer Herstellung zur Verfügung zu stellen.

Dementsprechend wurden neue, in 2-Stellung substituierte 1,5-Diaminopentane der allgemeinen Formel I

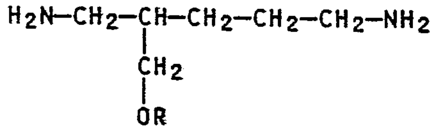

(I)

gefunden, in der R für einen $C_1$-$C_{20}$-Alkylrest, einen $C_3$-$C_{20}$-Oxoalkylrest, einen $C_2$-$C_{20}$-Alkenylrest, einen $C_5$-$C_{12}$-Cycloalkylrest oder einen $C_7$-$C_{20}$-Aralkylrest steht.

Diese Verbindungen sind dadurch erhältlich, daß man die entsprechenden Glutarsäuredinitrile der allgemeinen Formel II

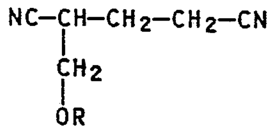

(II)

hydriert.

Bevorzugte Verbindungen (I) sind solche, in denen R für einen $C_1$-$C_{12}$-Alkylrest, einen $C_3$-$C_{12}$-Oxaalkylrest, einen $C_2$-$C_{12}$-Alkenylrest, einen $C_6$-$C_{12}$-Cycloalkylrest oder einen $C_7$-$C_{12}$-Aralkylrest steht. Besonders bevorzugt sind Verbindungen (I), in denen R eine Methyl-, Ethyl-, n-Propyl-, i-Propyl-, n-Butyl-, i-Butyl-, sec.-Butyl-, 2-Methylbutyl-, n-Pentyl-, Neopentyl-, 2-Methylpentyl-, n-Hexyl-, 2-Ethylhexyl-, n-Octyl-, n-Decyl-, n-Dodecyl-, Cyclohexyl-, Benzyl-, Phenylethyl-, Methoxyethyl-, Ethoxy-ethyl-, Butoxyethyl-oder Isobutoxyethylgruppe bedeutet, also z.B. das

2-Methoxymethyl-1,5-diaminopentan
2-Ethoxymethyl-1,5-diaminopentan
2-n-Propoxymethyl-1,5-diaminopentan
2-Isopropoxymethyl-1,5-diaminopentan
2-n-Butoxymethyl-1,5-diaminopentan
2-2-Methylbutoxymethyl-1,5-diaminopentan
2-Neopentoxymethyl-1,5-diaminopentan
2-2-Methylpentoxymethyl-1,5-diaminopentan
2-n-Hexoxymethyl-1,5-diaminopentan
2-2-Ethylhexoxymethyl-1,5-diaminopentan
2-n-Dodecoxymethyl-1,5-diaminopentan
2-n-Cyclohexoxymethyl-1,5-diaminopentan
2-(2-Methoxy)-ethoxymethyl-1,5-diaminopentan
2-(2-Ethoxy)-ethoxymethyl-1,5-diaminopentan
2-(2-Butoxy)-ethoxymethyl-1,5-diaminopentan und
2-(2-Isobutoxy)-ethoxymethyl-1,5-diaminopentan

Gegenstand der Erfindung ist auch die Verwendung der Diamine (I) zur Herstellung von 1,5-Diisocyanaten, indem man die Diamine in an sich bekannter Weise, z.B. mit Phosgen, in die Diisocyanate überführt.

Die Hydrierung der Dinitrile der allgemeinen Formel II zu den Diaminen der allgemeinen Formel I kann in an sich bekannter Weise erfolgen, z.B. durch katalytische Hyrierung, durch Umsetzen mit Lithiumaluminiumhydrid in inerten trockenen Lösungsmitteln oder mit Natrium in Gegenwart von Alkoholen; bevorzugt ist die katalytische Hydrierung an Katalysatoren aus der 8. Nebengruppe des Periodensystems.

Nach einer bevorzugten Ausführungsform des erfindungsgemäßen Verfahrens hydriert man die Verbindungen II, gegebenenfalls in einem inerten Lösungsmittel, in Gegenwart von Wasserstoff und Ammoniak bei einer Temperatur zwischen 50 und 200°C in Gegenwart eines Hydrierkatalysators mit einem Metall der 8. Nebengruppe des Periodensystems unter einem Druck von 1 bis 300 bar.

Ammoniak sollte zweckmäßig stöchiometrisch oder bevorzugt im Überschuß vorliegen, z.B. im Molverhältnis von 2:1 bis 40:1, besonders 5:1 bis 25:1, bezogen auf das eingesetzte Dinitril.

Die Reaktion kann diskontinuierlich, aber auch kontinuierlich durchgeführt werden. Als Reaktoren sind z.B. einfache Stahlautoklaven geeignet für kontinuierliche Hydrierungen druckfeste, mit Festbettkatalysatoren gefüllte Stahlrohre.

Als Hydrierkatalysatoren eignen sich alle für Nitrilhydrierungen üblichen Katalysatoren, bevorzugt aus der 8. Nebengruppe des Periodensystems, wie z.B. Nickel-, Kobalt-oder Eisen-Katalysatoren, aber auch Edelmetallkatalysatoren wie Palladium, Platin, Ruthenium oder Rhodium. Die Katalysatormetalle können als Vollkatalysatoren, z.B. in feiner Verteilung wie Raney-Nickel oder Raney-Kobalt in Suspensionsfahrweise als geformte metallische Eisenpigmentkatalysatoren, als Mischkatalysatoren oder auf Trägern niedergeschlagen, angewandt werden. Als Träger eignen sich beispielsweise Aluminiumoxid, Kieselgel oder Magnesiumsilikate. Besonders bevorzugt sind Raney-Kobalt und Eisenpigment-Katalysatoren.

Der Katalysator wird bei diskontinuierlicher Fahrweise zweckmäßig in Mengen von 1 bis 100 Gew.%, bevorzugt 10 bis 50 Gew.%, bezogen auf die Menge von (II), eingesetzt.

Als Lösungsmittel für die katalytische Hydrierung können alle unter den Reaktionsbedingungen inerten Lösungsmittel verwendet werden, vorzugsweise Tetrahydrofuran, Dioxan und Alkohole wie Methanol, Ethanol, Propanol oder Butanol oder Gemische dieser Lösungen. Auch flüssiges Ammoniak eignet sich als Lösungsmittel. Die Menge dieser Lösungsmittel beträgt im allgemeinen 1 bis 100, vorzugsweise 2 bis 20 kg pro kg (II).

Andere Methoden zur Nitrilhydrierung sind ebenso geeignet, z.B. die Reduktion mit komplexen Aluminiumhydriden (Lithiumaluminiumhydrid) in wasserfreien inerten Lösungsmitteln wie Ether oder Tetrahydrofuran, oder die Reduktion mit Natrium in Alkoholen wie z.B. in Ethanol, Propanol oder n-Butanol. Die Wahl der Reduktionsmethode kann u.U. von der Art des Restes R in den Verbindungen (II) abhängen. Katalytische Hydrierungen empfehlen sich beispielsweise besonders, wenn der Rest R gesättigt ist.

Die Aufarbeitung erfolgt in üblicher Weise, im Falle der katalytischen Hydrierung z.B. durch Abfiltrieren des Katalysators und Destillation oder Kristallisaton des Rückstandes.

Die Verbindungen (II) sind bekannt oder nach bekannten Methoden leicht zugänglich, z.B. aus 2-Methylenglutarsäuredinitril und Alkoholen R-OH gemäß der DE-A-15 93 176.

Die Diamine (I) sind wertvolle Zwischenprodukte, z.B. zur Herstellung der entsprechenden Diisocyanate. Durch Auswahl des Reste R können die Eigenschaften dieser Diamine und auch der Folgeprodukte wie der Diisocyanate, z.B. der Siedepunkt, die Flüchtigkeit, die Polarität und das Löslichkeitsverhälten, gezielt eingestellt werden. Weiterhin können die Diamine in Polyurethansystemen, in Polyamiden und in Epoxiharzen als Vernetzer eingesetzt werden.

Beispiel 1

2-Butoxydmethyl-1,5-diaminopentan

In einem Edelstahlautoklaven von 300 ml Inhalt wurden 12,5 g 2-Butoxymethyl-glutarsäuredinitril, 70 ml Tetrahydrofuran und 10 g Raney-Kobalt vorgelegt und gerührt. Nach Aufpressen von 50 ml flüssigem Ammoniak wurde der Druck mit Wasserstoff auf 140 bar erhöht, und der Autoklav wurde auf 110°C aufgeheizt. Durch Nachpressen wurde der Wasserstoffdruck sodann auf 160 bar gehalten. Nach 10 Stunden wurde abgekühlt und entspannt und der Reaktionsaustrag gaschromatographisch analysiert. Hiernach hatte sich das Dinitril vollständig umgesetzt und die Ausbeute am Diamin betrug 85 %. Durch fraktionierende Destillation wurde die reine Verbindung hergestellt; Siedepunkt 84 bis 86°C/0,3 mbar.

Beispiel 2

2-Hexoxymethyl-1,5-diaminopentan

120 g 2-Hexoxymethyl-glutarsäuredinitril 900 ml Tetrahydrofuran und 70 g Raney-Kobalt wurden in einem Edelstahlautoklaven mit 200 ml flüssigem Ammoniak vereinigt und unter Rühren und unter Wasserstoff 5 Stunden auf 110°C und 10 h auf 120°C erhitzt. Durch Nachpressen von Wasserstoff wurde der Druck zwischen 150 und 170 bar gehalten. Der Umsatz war praktisch vollständig, und die Ausbeute an dem Diamin betrug 76 %. Siedepunkt des reinen Diamins: 110 bis 115°C/0,6 mm.

Beispiel 3

2-Butoxymethyl-1,5-diaminopentan

Eine Rohrreaktor wurde mit 178 ml eines Eisenkatalysators, der nach DE-A 24 29 293 durch Reduktion von Eisenoxyden mit Wasserstoff bei einer Temperatur von etwa 500°C erhalten worden war, gefüllt. In Rieselfahrweise wurden dann stündlich 10 g 2-(Butoxymethyl)-glutarsäuredinitril, 90 g Tetrahydrofuran, 6,5 g flüssiges Ammoniak, 10 l rohes Hydriergemisch (Kreislauf-Fahrweise) sowie Wasserstoff zugeführt. Hydriert wurde bei einer Temperatur von 160°C und einem Druck von 100 bar. Die Ausbeute an Diamin betrug 74 % der Theorie.

Beispiel 4

2-(2-Methoxy)-ethoxymethyl-1,5-diaminopentan

91 g 2-(2-Methoxy)-ethoxymethyl-glutarsäuredinitril, 800 ml Tetrahydrofuran, 90 g Raney-Kobalt und 200 ml flüssiges Ammoniak wurden in einem Edelstahlautoklaven unter Rühren und unter Wasserstoff 5 h auf 100°C und 10 h auf 120°C erhitzt. Durch Nachpressen von Wasserstoff wurde der Druck auf 150 bar konstant gehalten. Die Ausbeute an dem Diamin betrug gemäß GC-Analyse 68 %; Siedepunkt 105-107°C/0,3 mbar.

**Ansprüche**

1. In 2-Stellung substituierte 1,5 Diaminopentane der allgemeinen Formel I

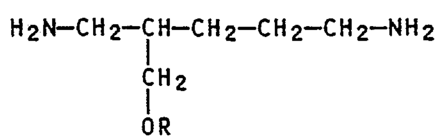

(I)

in der R für einen $C_1$-$C_{20}$-Alkylrest, einen $C_3$-$C_{20}$-Oxoalkylrest, einen $C_2$-$C_{20}$-Alkenylrest, einen $C_5$-$C_{12}$-Cycloalkylrest oder einen $C_7$-$C_{20}$-Aralkylrest steht.

2. In 2-Stellung substituierte 1,5-Diaminopentane der allgemeinen Formel I gemäß Anspruch 1, in der R für einen $C_1$-$C_{12}$-Alkylrest, einen $C_3$-$C_{12}$-Oxaalkylrest, einen $C_2$-$C_{12}$-Alkenylrest, einen $C_6$-$C_2$-Cycloalkylrest oder einen $C_7$-$C_{12}$-Aralkylrest steht.

3. In 2-Stellung substituierte 1,5-Diaminopentane der allgemeinen Formel I gemäß Anspruch 1, in der R eine Methyl-, Ethyl-, n-Propyl-, i-Propyl-, n-Butyl-, i-Butyl-, sec.-Butyl-, 2-Methylbutyl-, n-Pentyl-, Neopentyl-, 2-Methylpentyl-, n-Hexyl-, 2-Ethylhexyl-, n-Octyl-, n-Decyl-, n-Dodecyl-, Cyclohexyl-, Benzyl-, Phenylethyl-, Methoxyethyl-, Ethoxyethyl-, Butoxyethyl-oder Isobutoxyethylgruppe bedeutet.

4. Verfahren zur Herstellung der in 2-Stellung substituierten 1,5-Diaminopentane (I), dadurch gekennzeichnet, daß man ein Glutarsäuredinitril der allgemeinen Formel II

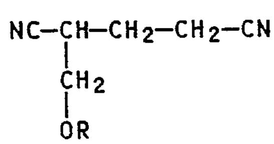

(II)

hydriert.

5. Verfahren nach Anspruch 4, dadurch gekennzeichnet, daß man die Hydrierung bei 50 bis 200°C und 1 bis 300 bar in einem inerten Lösungsmittel mit Wasserstoff und in Gegenwart von Ammoniak mittels eines Metalls der 8. Nebengruppe des Periodensystems katalytisch vornimmt.

6. Verfahren nach Anspruch 5, dadurch gekennzeichnet, daß man als Katalysator Raney-Kobalt oder einen Eisenpigmentkatalysator verwendet.

7. Verwendung der in 2-Stellung substituierten 1,5-Diaminopentane (I) zur Herstellung der entsprechenden Diisocyanate.

Europäisches Patentamt

# EUROPÄISCHER RECHERCHENBERICHT

Nummer der Anmeldung

EP 87113690.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP - A1 - 0 125 092 (ICI)<br>* Seite 16, Zeile 6 - Seite 17, Zeile 2; Seite 21, Zeilen 1,2 *<br>-- | 1-4 | C 07 C 93/04<br>C 07 C 119/042<br>C 07 C 118/00 |
| A | DE - A1 - 2 746 751 (SUN OIL COMPANY)<br>* Ansprüche 1,3,4 *<br>-- | 4,5 | |
| A | US - A - 3 631 198 (HORVITZ et al.)<br>* Ansprüche *<br>---- | 7 | |
| | | | RECHERCHIERTE SACHGEBIETE (Int. Cl.4) |
| | | | C 07 C 93/00<br>C 07 C 119/00<br>C 07 C 118/00 |

Der vorliegende Recherchenbericht wurde für alle Patentansprüche erstellt.

| Recherchenort | Abschlußdatum der Recherche | Prüfer |
|---|---|---|
| WIEN | 07-12-1987 | KÖRBER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument